(19) Europäisches Patentamt
European Patent Office
Office européen des brevets

(11) Numéro de publication: **0 296 975**
**A1**

(12) # DEMANDE DE BREVET EUROPEEN

(21) Numéro de dépôt: **88401580.1**

(22) Date de dépôt: **23.06.88**

(51) Int. Cl.4: **C 07 D 405/04**
C 07 F 9/65, A 61 K 31/495,
A 61 K 31/50

(30) Priorité: **23.06.87 FR 8708830**
**12.01.88 FR 8800278**
**09.03.88 FR 8803064**

(43) Date de publication de la demande:
**28.12.88 Bulletin 88/52**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Garcia, Georges**
**27, rue des Aires**
**F-34980 - Saint Gely du Fesc (FR)**

**Roux, Richard**
**9, Lotissement de l'Arnède**
**F-34570 - Vailhauques (FR)**

**Nisato, Dino**
**2, rue de Terre Rouge**
**F-34680 - Saint Georges d'Orques (FR)**

**Gautier, Patrick**
**Lieu dit Manavieille**
**F-34660 - Cournonterral (FR)**

(74) Mandataire: **Gillard, Marie-Louise et al**
**Cabinet Beau de Loménie 55, Rue d'Amsterdam**
**F-75008 Paris (FR)**

Revendications pour les Etats contractants suivants: ES + GR.

(54) **Dérivés du diméthyl-2,2 chromannol-3, procédé d'obtention et compositions pharmaceutiques les contenant.**

(57) L'invention a pour objet des dérivés du diméthyl-2,2 chromannol-3 de formule :

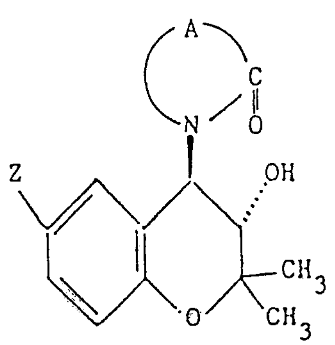

(I)

dans laquelle

- A représente, entre N et CO, le groupe - CH = CH - E = CH - ou le groupe

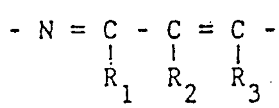

;

- Z représente un halogène ou un groupe cyano, acétyle, trifluoroacétyle, nitro, alkylthio, carboxy, phosphono, dialcoxyphosphoryle, alcoxycarbonyle, les groupes alkylthio et alcoxy contenant de 1 à 3 atomes de carbone;
- E représente un atome d'azote ou un groupe C ($R_4$);
- $R_1$ représente l'hydrogène, un groupe méthyle ou un groupe hydroxyle, $R_2$ et $R_3$ représentant chacun indépendamment l'hydrogène ou un groupe méthyle, un seul des substituants $R_1$, $R_2$ $R_3$ pouvant être méthyle;
- $R_4$ représente un atome d'hydrogène, d'halogène, un groupe méthyle ou un groupe hydroxyle ;

et les sels pharmaceutiquement acceptables du groupe phosphono ou carboxy.

Ces dérivés ont une activité antiarythmique et antihypertensive.

EP 0 296 975 A1

## Description

**Dérivés du diméthyl-2,2 chromannol-3 , procédé d'obtention et compositions pharmaceutiques les contenant.**

La présente invention a pour objet des dérivés du chromanne à activités antihypertensive et antiarythmique.

Le brevet belge 829 611 mentionne toute une série de dérivés du chromannol-3 à activité antihypertensive ; ces dérivés sont caractérisés par la présence en position 4 d'un groupe $NR_1R_2$ dans lequel $R_1$ est l'hydrogène ou un groupe hydrocarboné éventuellement substitué, $R_2$ est l'hydrogène ou un alkyle, $NR_1R_2$ pouvant être un groupe hétérocyclique comportant de 3 à 8 atomes, non substitué ou substitué par un ou deux groupes méthyle et par la présence éventuelle d'un grand nombre de substituants possibles en position 6 ou en position 7.

La demande de brevet européen publiée sous le numéro 76 075 décrit des dérivés du chromannol-3 à activité antihypertensive caractérisés par la présence en position 4 d'un groupe oxo-2 pyrrolidinyl-1 ou d'un groupe oxo-2 pipéridinyl-1 et par la présence éventuelle de nombreux substituants possibles, dont le groupe cyano, en position 6 ou en position 7.

On a maintenant trouvé que les dérivés du diméthyl-2,2 chromannol-3 caractérisés par la présence, en position 4, d'un atome d'azote appartenant à un hétérocycle à 6 chaînons comportant 2 doubles liaisons conjuguées avec un groupe oxo, possèdent une excellente activité antihypertensive et antiarythmique et une toxicité très faible.

Ainsi la présente invention concerne, selon un de ses aspects, des dérivés du trans-diméthyl-2,2 chromannol-3 de formule :

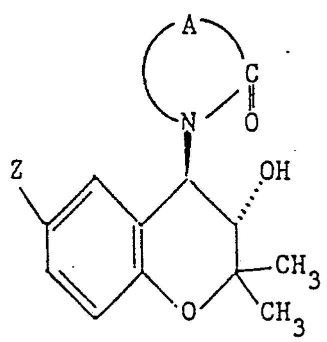

(I)

dans laquelle

- A représente, entre N et CO, le groupe - CH = CH - E = CH - ou le groupe

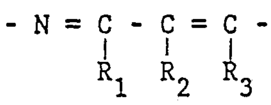

;

- Z représente un halogène ou un groupe cyano, acétyle, trifluoroacétyle, nitro, alkylthio, carboxy, phosphono, dialcoxyphosphoryle, alcoxycarbonyle, les groupes alkylthio et alcoxy contenant de 1 à 3 atomes de carbone;
- E représente un atome d'azote ou un groupe C ($R_4$);
- $R_1$ représente l'hydrogène, un groupe méthyle ou un groupe hydroxyle, $R_2$ et $R_3$ représentent chacun indépendamment l'hydrogène ou un groupe méthyle, un seul des substituants $R_1$ $R_2$, $R_3$ pouvant être méthyle;
- $R_4$ représente un atome d'hydrogène, d'halogène, un groupe méthyle ou un groupe hydroxyle;

et les sels pharmaceutiquement acceptables du groupe phosphono ou carboxy.

Dans la présente description et dans las revendications qui vont suivre, on entend par halogène un atome de fluor, de chlore ou de brome.

Les sels pharmaceutiquement acceptables sont de préférence ceux des métaux alcalins et alcalino-terreux tels que les sels de sodium et de potassium ou ceux des bases organiques comme la triéthanolamine, le trométamol, l'éthanolamine,la N-méthylpipéridine ou la tert-butylamine.

Les composés (I) selon l'invention présentent deux carbones asymétriques en positions 3 et 4 du noyau chromanne. La présente invention comprend chacun des stéréoisomères de I ainsi que le racémique.

La présente invention a également pour objet un procédé pour la préparation des composés (I).

Ledit procédé est caractérisé en ce que l'on traite un époxyde de chromanne de formule :

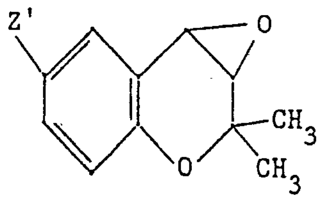

(II)

dans laquelle Z′ a la même signification donnée ci-dessus à Z, mais n'est pas le groupe carboxy ou le groupe phosphono, avec un hétérocycle répondant à l'une des formes tautomères suivantes :

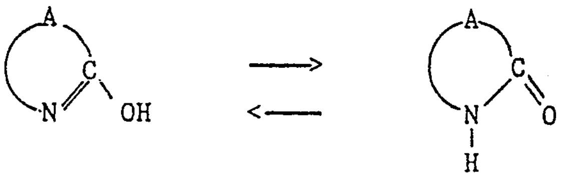

(III)

dans lesquelles A, entre N et CO ou C(OH), a la signification indiquée précédemment pour (I); on transforme éventuellement le groupe alcoxycarbonyle en groupe carboxy ou le groupe dialcoxyphosphoryle en groupe phosphono ; et on transforme éventuellement l'acide carboxylique ou l'acide phosphonique ainsi obtenu en l'un de leurs sels pharmaceutiquement acceptables.

La réaction d'ouverture de l'époxyde (II) est conduite à une température comprise entre 10°C et 100°C dans une solvant organique inerte comme le dioxanne, le tétrahydrofuranne, le méthyl-tert-butyléther, le diméthylsulfoxyde ou le diméthylformamide en présence d'un agent de condensation basique tel que l'hydrure de sodium, un hydroxyde d'ammonium quaternaire comme l'hydroxyde de benzyltriméthylammonium. Dans ces conditions opératoires, l'ouverture de l'époxyde (II) conduit à des composés de formule I ayant la configuration trans.

A la fin de la réaction on obtient un composé de formule :

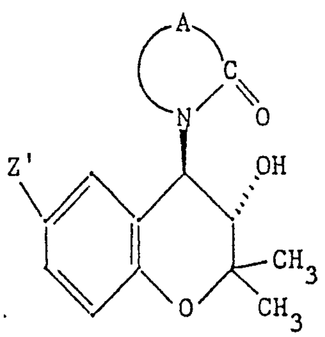

(Ia)

dans laquelle A et Z′ sont tels que définis ci-dessus, qui est isolé selon les méthodes classiques.

Lorsque Z′ représente le groupe alcoxycarbonyle, la transformation en groupe carboxy est effectuée par des méthodes connues.

Lorque Z′ représente un groupe dialcoxyphosphoryle, celui-ci peut être transformé en groupe phosphono correspondant par transestérification avec un halogénure de triméthylsilyle, de préférence le bromure, et hydrolyse du di(triméthylsilylester) par simple action de l'eau. On obtient ainsi un composé de formule I, dans laquelle Z représente un groupe carboxy ou phosphono, et ledit composé peut être transformé en l'un de ses sels pharmaceutiquement acceptables, par exemple ceux de métaux alcalins ou alcalino-terreux tels que les sels de sodium ou de potassium ou ceux des bases organiques comme la triéthanolamine, le trométamol, l'éthanolamine, la tert butylamine ou la N-méthylpipéridine.

Les époxydes de départ de formule II sont connus ou préparés selon des méthodes connues. Ainsi l'époxyde (II) dans lequel Z′ représente le groupe cyano est décrit dans le brevet belge 852 955 ; les époxydes (II) dans lesquels Z′ représente le groupe nitro, le groupe acétyle ou un groupe alcoxycarbonyle sont décrits dans J. Med. Chem., 1983, 26, 1582-1589 ; les époxydes (II) dans lesquels Z′ représente un groupe alkylthio sont préparés selon une méthode analogue ; les époxydes (II) dans lesquels Z′ représente un halogène sont préparés selon Tetrahedron, 1981, 37, (15), 2613-2616.

Les composés de formule II dans laquelle Z′ représente un groupe trifluoroacétyle ou un groupe dialcoxyphosphoryle ne sont pas décrits en littérature Ces composés sont préparés à partir de la chromène

correspondante de formule:

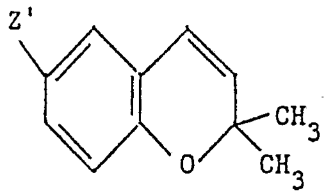

(IV)

sur laquelle on fait agir la N-bromosuccinimide dans du diméthylsulfoxide aqueux pour obtenir le dérivé bromé de formule:

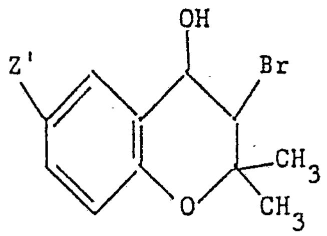

(V)

Ce composé (V) est traité avec un agent alcalin dans un mélange eau/solvant organique, par exemple eau/dioxanne, de préférence à la température ambiante, pendant une période de 8 à 20 heures et l'époxyde ainsi obtenu de formule II est isolé selon des méthodes classiques, par exemple par concentration du mélange réactionnel et récupération du résidu avec un solvant qui élimine les impuretés, tel que le chlorure de méthylène, lavage à l'eau et concentration.

Lorsque Z' représente un groupe dialcoxyphosphoryle, la chromène IV peut être préparée à partir de la bromo-6, diméthyl-2,2 chromène (J Chem. Soc. 1960, 3094 3098) de formule :

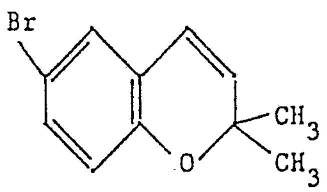

(VI)

par action d'un trialkylphosphite, en présence de chlorure de Nickel à 180°C.

Lorsque Z' représente le groupe trifluoroacétyle, la chromène (IV) peut être préparée à partir du trifluoroacétyl-4 phénol (J. Msd. Chem., 1965, 8, 229) par addition en milieu basique, en présence d'un catalyseur de transfert de phase, de chloro-3 méthyl 3 butyne-1.

Les hétérocycles de formule (III) sont connus et disponibles dans le commerce ou sont préparés selon des méthodes connues.

Les composés selon l'invention augmentent la polarisation des fibres musculaires lisses et ont un effet vasodilatateur sur la veine porte ; leur effet antihypertenseur a été observé chez l'animal.

Par ailleurs, on a observé que les composés selon l'invention accélèrent la repolarisation des cellules myocardiques; parallèlement, leur effet antiarythmique a été observé sur un modèle animal.

Aucun signe de toxicité n'est observé avec ces composés aux doses pharmacologiquement actives.

Ainsi les composés selon l'invention peuvent être utilisés dans le traitement de l'hypertension,des troubles pathologiques associés aux contractions des fibres musculaires lisses des appareils gastrointestinal, respiratoire, utérin et urinaire, par exemple: ulcère, asthme, contraction utérine prématurée, incontinence, et dans le traitement d'autres troubles pathologiques cardiovasculaires tels que : angor, insuffisance cardiaque, maladies vasculaires cérébrales et périphériques. De plus, les composés selon l'invention peuvent être utilisés dans le traitement de l'arythmie cardiaque. Enfin, les composés de la présente invention peuvent être utilisés pour le traitement topique de l'alopécie.

La présente invention a également pour objet des compositions pharmaceutiques contenant une dose efficace d'un composé selon l'invention et des excipients convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, transdermique ou rectale, les principes actifs de

formule I ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale.

Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 5 mg par kg de poids du corps et par jour.

Chaque dose unitaire peut contenir de 0,5 à 200 mg, de préférence de 1 à 50 mg, d'ingrédients actifs en combinaison avec un support pharmaceutique Cette dose unitaire peut être administrée 1 à 5 fois par jour de façon à administrer un dosage journalier de 0,5 à 1000 mg, de préférence de 1 à 250 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs tels que, par exem ple, des tranquillisants ou d'autres médicaments qui peuvent être utiles dans le traitement des troubles ou maladies indiquées ci-dessus.

Les exemples suivants illustrent l'invention sans toutefois la limiter. Dans les exemples, ainsi que dans la partie descriptive et dans les revendications, les produits sont désignés comme dérivés du chromanne. Il est entendu que les produits de la présente invention sont des dérivés du diméthyl-2,2 dihydro-3,4 2H-chromène et que le terme "chromanne" désigne le "dihydro-3,4 2H-chromène".

PREPARATION I

a) Diéthylphosphono-6 diméthyl-2,2 2H-chromène

16 g de bromo-6 diméthyl-2,2 2H-chromène sont dissous dans 100 ml de triéthylphosphite. On ajoute 2 g de chlorure de nickel et on chauffe à 180° C pendant 24 heures dans un autoclave. Après avoir concentré le triéthylphosphite restant, le produit attendu distille à 130-140° C sous 133,322 Pa. On recueille 11,5 g.

b) trans Bromo-3 diéthylphosphono-6 diméthyl 2,2 chromannol-4

11 g du produit précédent sont dissous dans 62 ml de diméthylsulfoxide contenant 1,35 ml d'eau. En maintenant la solution à une température inférieure à 20° C, on ajoute par petites fractions 12,2 g de N-bromosuccinimide. On laisse sous agitation à température ambiante pendant 30 minutes puis on ajoute 100 ml d'eau et on extrait à l'acétate d'éthyle.Après séchage sur sulfate de sodium, on concentre la phase organique, reprend le résidu par 100 ml d'acétone et 50 ml d'eau, puis on chauffe au reflux pendant 5 heures On concentre l'acétone, extrait à l'éther, sèche sur sulfate de sodium et concentre. Le produit attendu cristallise dans l'éther isopropylique. Après filtration et séchage des cristaux on recueille 3,2 g de produit. Point de fusion : 124° C.

c) Diéthylphosphono-6 diméthyl-2,2 époxy-3,4 chromanne

On mélange 23 g du composé obtenu à l'étape précédente et 12 g de soude dans 900 ml de dioxanne et 100 ml d'eau. Après 24 heures à température ambiante, on concentre le dioxanne, on reprend le résidu par de l'eau et extrait à l'éther éthylique puis sèche sur sulfate de sodium. Après concentration, on obtient 16,2 g du produit attendu.

PREPARATION II

a) Diméthyl-2,2 trifluoroacétyl-6 chromène.

On porte à reflux, sous agitation, pendant 48 heures un mélange contenant: 8,4 g de trifluoroacétyl-4 phénol (préparé selon J. Med Chem., 1965, 8, 229), 1,8 g de soude en pastilles, 50 ml de chlorure de méthylène,30 ml d'eau, 11 g d'une solution méthanolique à 35 % d'hydroxyde de benzyl-triméthylammonium, 14 ml de tris (dioxa-3,6 heptyl)amine, et 6,1 g de chloro-3 méthyl-3 butyne-1. On ajoute 11 g de chloro-3 méthyl-3 butyne et l'on maintient le mélange au reflux pendant 4 jours. On ajoute ensuite 50 ml d'eau, 100 ml de chlorure de méthylène, on laisse décanter, on lave la phase organique par de la soude 1 N puis de l'eau, on sèche, puis on concentre sous vide pour obtenir 10 g d'une huile utilisée brute pour la cyclisation On ajoute 40 ml de dichloro-l,2 benzène et l'on porte à reflux pendant 2 heures. On concentre sous vide puis on reprend le résidu dans 40 ml d'héxane et l'on effectue une chromatographie sur une colonne de 300 g de silice en éluant par l'éther isopropylique. On obtient 3,5 g d'une huile jaune.

Rendement : 40 %

CCM sur silice (héxane-acétone : 7-3) : Rf = 0,66

Les spectres IR et RMN confirment la structure.

b) Bromo-3 diméthyl-2,2 hydroxy-4 trifluoroacétyl-6 chromanne.

On mélange, sous agitation à 15° C, 3,4 g de diméthyl-2,2 trifluoroacétyl-6 chromène, préparé à l'étape précédente, dans 30 ml de diméthylsulfoxide et 5 ml d'eau. On ajoute en 20 minutes 5 g de N-bromosuccinimide et on laisse sous agitation pendant 2 heures. On verse le mélange dans l'eau glacée, filtre le précipité formé, lave à l'eau puis sèche. Après dissolution dans 300 ml d'héxane, on sépare l'insoluble par filtration et concentre le filtrat. On obtient 3 g d'un solide jaunâtre.

Rendement : 68 %

Point de fusion : 103° C

CCM sur silice (héxane-acétone : 7-3) : Rf = 0,55

Les spectres IR et RMN confirment la structure.

c) Diméthyl-2,2 époxy-3,4 trifluoroacétyl-6 chromanne.

On laisse sous agitation à 20° C pendant 20 heures un mélange contenant 2,9 g de bromo-3 diméthyl-2,2 hydroxy-4 trifluoroacétyl-6 chromanne préparé à l'étape précédente, 25 ml de dioxanne, 0,5 g de soude en pastilles et 5 ml d'eau. Après avoir concentré sous vide à 40° C, on traite le résidu 4 fois par 100 ml d'éther éthylique puis on sèche et concentre sous vide. On obtient 2 g d'une huile jaune. Rendement : 90 %

CCM sur silice (dichlorométhane) : Rf = 0,80

Les spectres IR et RMN confirment la structure.

PREPARATION III

La fluoro-4 pyridone-2 est préparée selon C.C. Leznoff et al., J. Het. Chem., 1985, 22, p.145 de la façon suivante:

a) Chlorhydrate de méthoxy-2 pyridine N-oxyde

Ce composé est obtenu à partir de 21,8 g de méthoxy-2 pyridine et de 144,4 g d'acide méta-chloro perbenzoïque aqueux à 45 % dans 500 ml de dichlorométhane

Poids : 23,6 g

Rendement : 61 %

Point de fusion : 123° C

b) Méthoxy-2 nitro-4 pyridine N-oxyde.

A 15,5 g du chlorhydrate de méthoxy-2 pyridine N-oxyde, on ajoute 30 ml d'acide sulfurique concentré refroidi à 0° C puis on introduit goutte à goutte, à 0° C, 42 ml d'acide nitrique fumant et 14 ml d'acide sulfurique concentré. Après avoir laissé remonter la température, on chauffe pendant 3 heures à 90° - 100° C puis le mélange réactionnel est refroidi et versé sur 50 g de glace. On neutralise à pH 7 par addition d'ammoniaque concentré à une température inférieure à 10° C. La phase aqueuse est extraite 6 fois par du dichlorométhane puis la phase organique est séchée sur sulfate de sodium et concentrée, on récupère 11 g d'un solide jaune correspondant au mélange de méthoxy-2 nitro-4 pyridine N-oxyde et méthoxy-2 nitro-5 pyridine N-oxyde. On sépare les 2 produits par chromatographie sur silice en éluant par le mélange dichlorométhane méthanol (95-5). On receuille 6,3 g de méthoxy-2 nitro-4 pyridine N-oxyde.

Point de fusion : 180° C

Rendement : 46,3 %.

c) Amino-4 méthoxy-2 pyridine.

On chauffe 1 heure à 100° C un mélange de 6,3 g de méthoxy-2 nitro-4 pyridine N-oxyde et 12,6 g de fer en poudre dans 150 ml d'acide acétique. Après refroidissement, on ajoute de la soude puis on filtre et lave à l'eau. On extrait la phase aqueuse par l'éther éthylique. Après séchage et concentration, on récupère 3,6 g d'une huile qui cristallise en solide blanc.

Rendement : 78,4 %

Point de fusion : 88° C.

d) Fluoro-4 méthoxy-2 pyridine.

On refroidit à 10°C une solution contenant 3,6 g d'amino 4 méthoxy-2 pyridine dans 15 ml d'acide fluoroborique à 48 % On ajoute par petites portions à une température inférieure à -5°C, 3 g de nitrite de sodium puis on laisse sous agitation pendant 45 minutes à 0°C. On amène lentement à température ambiante puis laisse sous agitation dans un bain d'eau pendant 30 minutes. On refroidit à -10°C puis neutralise en ajoutant de la soude 3 M en maintenant à basse température. Le mélange est extrait par 50 ml d'éther éthylique, on lave la phase organique par 5 ml d'eau froide et sèche sur fluorure de potassium anhydre. L'éther est distillé à pression atmosphérique. Le résidu huileux est utilisé tel quel à l'étape suivante.

e) Fluoro-4 pyridone-2.

A une solution de 3,3 g de produit obtenu précédemment dans 13 ml de dichlorométhane, on ajoute, goutte à goutte, sous Argon, 5,85 g d'iodotriméthylsilane dans 3 ml de dichlorométhane. On laisse sous agitation à température ambiante pendant 4 heures puis on porte à 40°C pendant 1 heure et laisse une nuit à température ambiante. On concentre le solvant sous vide, ajoute 10 ml de dichlorométhane et concentre à nouveau sous vide. Le résidu obtenu est dissout dans du dichlorométhane, on ajoute quelques cristaux de thiosulfate de sodium et on laisse sous agitation jusqu'à ce que la coloration brune disparaisse. Le mélange est essoré, le filtrat est séché sur sulfate de sodium. Le résidu minéral est repris dans du méthanol puis essoré. Le résidu minéral est chromatographié sur silice en éluant par un mélange dichlorométhane-méthanol (9 : 1) On récupère 0,590 g d'un solide blanc.

Point de fusion : 172°C

EXEMPLE 1

trans-Cyano-6 (dihydro-1,2 oxo-2 pyridyl-1)-4 diméthyl-2,2 chromannol-3 : SR 44 709.

On chauffe à reflux pendant 40 heures 1 g de cyano-6 diméthyl-2,2 époxy-3,4 chromanne avec l g d'hydroxy-2 pyridine dans 10 ml de dioxanne en présence de 0,20 ml d'une solution méthanolique contenant 35 % d'hydroxyde de benzyltriméthyl ammonium. On reprend par 30 ml d'eau, le précipité obtenu et essoré, lavé par l'éther isopropylique puis recristallisé dans 20 ml d'alcool éthylique absolu. On obtient 0,9 g du produit attendu.

Point de fusion : 243°C avec décomposition (tube capillaire).

EXEMPLE 2

trans-Diéthylphosphono-6 (dihydro-1,2 oxo-2 pyridyl-1)-4 diméthyl-2,2 chromannol-3 : SR 44 745.

On chauffe à reflux pendant 48 heures un mélange contenant 5,6 g diéthyphosphono-6 diméthyl-2,2 époxy-3,4 chromanne (PREPARATION I), 2,5 g d'hydroxy-2 pyridine et 4 gouttes d'hydroxyde de benzyltriméthyl ammonium. Après avoir concentré le dioxanne, on reprend le résidu dans du dichlorométhane, lave deux fois à l'eau puis sèche sur sulfate de sodium et concentre à sec. On ajoute de l'éther éthylique, le produit cristallise. Après recristallisation dans l'acétate d'éthyle, on obtient 1,5 g du produit attendu.

Point de fusion : 130,5°C (tube capillaire).

EXEMPLE 3

trans-(Dihydro-1,2 oxo-2 pyridyl-1)-4 diméthyl-2,2 trifluoroacétyl-6 chromannol-3 : SR 45 160.

On porte à reflux pendant 20 heures un mélange contenant 1,9 g de diméthyl 2,2 époxy-3,4 trifluoroacétyl-6 chromanne (PREPARATION II), 1,4 g d'hydroxy-2 pyridine, 5 ml de dioxanne et 0,2 ml d'une solution méthanolique à 35% d'hydroxyde de benzyltriméthylammonium. Le résidu obtenu après concentration sous vide est repris par 20 ml d'eau puis on filtre l'insoluble, lave à l'eau et à l'éther isopropylique bouillant. On effectue une chromatographie sur une colonne de 50 g de silice en éluant par le mélange chlorure de méthylène - acétate d'éthyle (7-3). On obtient 0,3 g de produit sec.

Rendement : 12 %

Spectre IR :

1150 $cm^{-1}$ : C-0-C (chromanne)

1665 $cm^{-1}$ : CO (pyridone)

1720 $cm^{-1}$ : CO ($CF_3CO$)

3670 $cm^{-1}$ : OH

EXEMPLE 4

trans-cyano-6 (dihydro-1,2 hydroxy-4 oxo-2 pyridyl-1)-4 diméthyl-2,2 chromannol-3 : SR 44 793.

On chauffe à reflux pendant 20 heures 3 g de cyano-6 diméthyl-2,2 époxy-3,4 chromanne avec 1,8 g de dihydroxy-2,4 pyridine dans 30 ml de dioxanne et 20 ml de diméthylformamide en présence de 0,6 ml d'une solution méthanolique comprenant 35 % d'hydroxyde de benzyltriméthyl ammonium. On évapore les solvants sous vide puis on cristallise le résidu par addition d'éther isopropylique. Les cristaux obtenus sont repris par de l'eau, filtrés puis lavés à l'acétone. On obtient 850 mg du produit attendu.
Point de fusion : 248-250°C

EXEMPLE 5

trans-Cyano-6 (dihydro-1,6 oxo-6 pyridazinyl-1)-4 diméthyl-2,2 chromannol-3 : SR 44 758.

On chauffe à reflux pendant 20 heures 1 g de cyano 6 diméthyl-2,2 époxy-3,4 chromanne, 1 g d'hydroxy-3 pyridazine dans 5 ml de dioxanne en présence de 0,20 ml d'une solution méthanolique contenant 35 % d'hydroxyde de benzyltriméthylammonium On concentre sous vide puis on triture le résidu dans 20 ml d'eau ; le précipité obtenu est essoré, lavé à l'eau, à l'éther isopropylique puis on recristallise dans 10 ml d'alcool isopropylique. On obtient 0,9 g du produit attendu.
Point de fusion : 211-215°C - Rf/silice (acétate d'éthyle) : 0,34.

EXEMPLE 6

trans-Cyano-6 (dihydro-1,6 hydroxy-3 oxo-6 pyridazinyl-1)-4 diméthyl-2,2 chromannol-3 : SR 44 994.

On chauffe à reflux pendant 50 heures un mélange contenant 1 g de cyano-6 diméthyl-2,2 époxy-3,4 chromanne, 1,1 g de dihydroxy-3,6 pyridazine et 0,20 ml d'une solution méthanolique contenant 35 % d'hydroxyde de benzyltriméthylammonium, dans 5 ml de dioxanne et 5 ml de diméthylformamide. On concentre sous vide puis on triture le résidu dans 15 ml d'eau Le précipité formé est essoré, lavé à l'eau puis à l'éther isopropylique.
On cristallise dans 60 ml d'éthanol à 95 %. On obtient 0,7 g du produit attendu.
Point de fusion: 251-252°C - Rf/silice (acétate d'éthyle-éthanol, 8 : 2) = 0,63.

EXEMPLES 7 ET 8

trans-Cyano-6 (dihydro-1,6 hydroxy-3 méthyl-5 oxo-6 pyridazinyl-1)-4 diméthyl-2,2 chromannol-3: SR 45209 (Ex. 7).

trans-Cyano-6 (dihydro-1,6 hydroxy-3 méthyl-4 oxo-6 pyridazinyl-1)-4 diméthyl-2,2 chromannol-3: SR 45218 (Ex. 8).

On maintient à 120°C pendant 8 heures un mélange contenant 2 g de cyano-6 diméthyl-2,2 époxy-3,4 chromanne, 2,5 g de dihydroxy-3,6 méthyl-4 pyridazine, 30 ml de diméthylformamide et 0,4 ml d'une solu tion méthanolique contenant 35% d'hydroxyde de benzyltriméthylammonium.
On concentre sous vide puis on triture dans 30 ml d'eau, essore, lave par de l'éthanol absolu puis cristallise dans 40 ml de méthanol. On obtient 1,3 g d'un mélange de 2 produits que l'on sépare par chromatographie sur colonne de silice.
SR 45209: Rf (Acétate d'éthyle - Alcool éthylique : 8 - 2): 0,80
SR 45218: Rf (Acétate d'ethyle - Alcool éthylique : 8 - 2): 0,71
La structure des 2 produits est confirmée par l'analyse de leur spectre de RMN enregistré à 250 MHz dans le DMSO.
Les abréviations suivantes sont utilisées pour le décrire:
s = singulet
d = doublet
m = multiplet ou massif
J = constante de couplage.

SPECTRE DE RMN

SR 45209

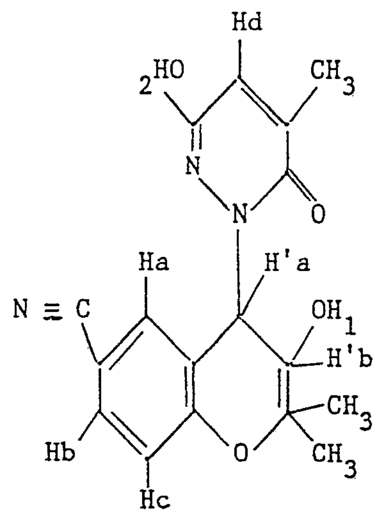

| Delta ppm | Aspect | Protons | Attribution |
|---|---|---|---|
| 1,25<br>1,40 | 2 s | 6 H | 2$CH_3$ |
| 2,09 | s | 3 H | $CH_3$ (pyridazine) |
| 3,80 | d de d<br>J = 6Hz,<br>J = 8Hz | 1 H | H'b |
| 5,87 | d,J = 8Hz | 1 H | H'a |
| 5,90 | d,J = 6Hz | 1 H | $OH_1$ |
| 6,95 | d,J = 10Hz | 1 H | Hc |

| | | | |
|---|---|---|---|
| 7,19 | s | 1 H | Hd |
| 7,67 | d de d<br>J = 10Hz,<br>J = 2Hz | 1 H | Hb |
| 7,73 | d,J = 2Hz | 1 H | Ha |
| 12 | s | 1 H | $OH_2$ |

SPECTRE DE RMN

SR 45218

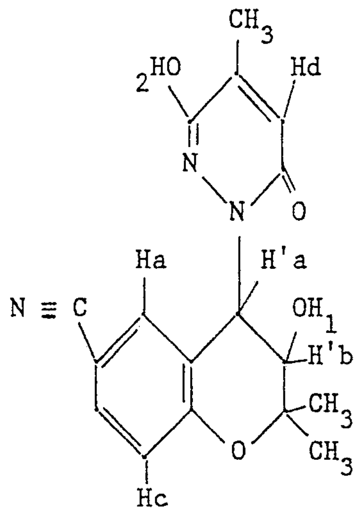

| Delta ppm | Aspect | Protons | Attribution |
|---|---|---|---|
| 1,29<br>1,40 | 2 s | 6 H | $2CH_3$ |
| 2,09 | s | 3 H | $CH_3$ (pyridazine) |
| 3,83 | d de d<br>J = 4Hz,<br>J = 6Hz | 1 H | H'b |
| 5,78 | d,J = 6Hz | 1 H | H'a |
| 5,90 | d,J = 4Hz | 1 H | $OH_1$ |
| 6,80 | s | 1 H | Hd |

| | | | | |
|---|---|---|---|---|
| 7,00 | d,J = 8Hz | 1 H | Hc | |
| 7,68 | d,J = 8Hz | 1 H | Hb | |
| 7,78 | s | 1 H | Ha | |
| 12,10< | s | 1 H | $OH_2$ | |
| >11,8 | élargi | | | |

EXEMPLE 9

trans-Cyano-6 (dihydro-1,6 méthyl-3 oxo-6 pyridazinyl-1)-4 diméthyl-2,2 chromannol-3: SR 45225.

On prépare un mélange contenant 1 g de cyano-6 diméthyl-2,2 époxy-3,4 chromanne, 1,1 g de méthyl-6 pyridazinone-3 (préparée selon la méthode décrite dans J. Chem. Soc., 1947, p. 241 par action du levulinate d'éthyle sur l'hydrate d'hydrazine) dans 10 ml de dioxanne contenant 0,1 ml d'une solution méthanolique à 35 % d'hydroxyde de benzyltriméthylammonium. Après 2 jours à température ambiante, on dilue à l'eau, essore, dissout dans l'acétate d'éthyle puis on décante l'eau, sèche et concentre. Après recristallisation dans un mélange acétate d'éthyle-éther isopropylique, on obtient 0,6 g du produit attendu.
Point de fusion : >257°C.
Le produit est caractérisé par son spectre de RMN enregistré à 250 MHz.

SPECTRE DE RMN

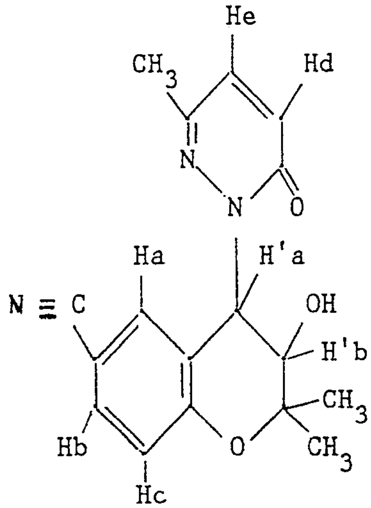

| Delta ppm | Aspect | Protons | Attribution |
|---|---|---|---|
| 1,25 | s | 6 H | $2CH_3$ |
| 1,46 | s | | |
| 2,2 | s | 3 H | $CH_3$ (pyridazine) |
| 4,15 | m | 1 H | H'b |
| 5,78 | d,J = 8Hz | 1 H | OH |
| 6 | m | 1 H | H'a |
| 6,96 à 7,68 | m | 5 H | Ha, Hb, Hc, Hd, He |

EXEMPLE 10

trans-Cyano-6 (dihydro-1,2 fluoro 4 oxo-2 pyridyl-1)-4 diméthyl-2,2 chromannol-3: SR 45311.

On porte à reflux pendant 24 heures un mélange contenant 460 mg de cyano-6 diméthyl-2,2 époxy-3,4 chromanne, 283 mg de fluoro-4 pyridone 2 (PREPARATION III) et 0,05 ml d'une solution méthanolique contenant 35 % d'hydroxyde de benzyltriméthylammonium dans 5 ml de tétrahydrofuranne On concentre le solvant et reprend dans l'acétate d'éthyle puis on lave la phase organique à l'eau, sèche sur sulfate de sodium et concentre sous vide. Le résidu obtenu est chromatographié sur silice en éluant par un mélange dichlorométhane-méthanol (97 : 3). On récupère 200 ml d'un solide blanc.
Point de fusion: 235° C.

En suivant des modes opératoires semblables, on a également préparé les composés selon l'invention rassemblés dans le tableau 1 ci-dessous.

TABLEAU 1

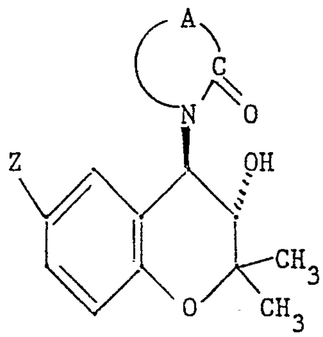

| Exemple N° | Produit N° | Z | 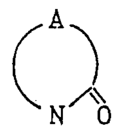 | Point de fusion (solvant de recristallisation) |
|---|---|---|---|---|
| 11 | SR 45012 | CN | 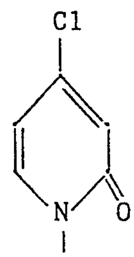 | 260 - 261°C (AcOEt) |
| 12 | SR 45067 | CN | 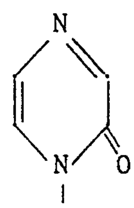 | 251 - 253°C (AcOEt - $CH_2Cl_2$) |
| 13 | SR 45135 | CN | 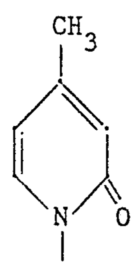 | 266 - 267°C (THF) |

| | | | | |
|---|---|---|---|---|
| 14 | SR 45373 | Br | 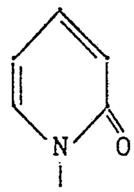 | 248°C ($(iPr)_2O$) |
| 15 | SR 45434 | Cl | 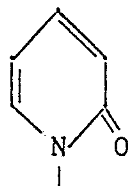 | 250°C (AcOEt) |
| 16 | SR 45484 | $CH_3CO$ | 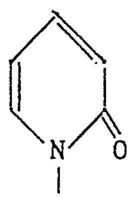 | 260°C (THF) |
| 17 | SR 45485 | $NO_2$ | 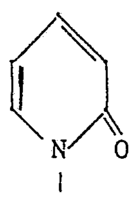 | 224 - 226°C (AcOEt) |
| 18 | SR 45512 | $CH_3S$ | 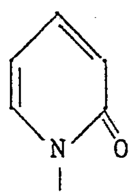 | 154°C |

Lorsque le produit a été recristallisé, le solvant de recristallisation est indiqué entre parenthèses. Les abréviations utilisées ont les significations suivants :
AcOEt : acétate d'éthyle
THF : tétrahydrofuranne
$(iPr)_2O$ : éther isopropylique.

Des compositions pharmaceutiques contenant le produit selon l'invention ont été préparées.

EXEMPLE 19

Comprime enrobé.

Des comprimés peuvent être préparés par granulation humide. On utilise comme solvant auxiliaire de fabrication l'alcool éthylique et l'eau purifiée. Après l'évaporation de ces solvants, le stéarate de magnésium intervient en phase externe comme lubrifiant. On procède ensuite à l'enrobage des comprimés.

Formulation
SR 44 709 : 1mg
Alcool éthylique à 95 % : 0,02 ml
Cellulose microcristalline : 48 mg
Lactose : 69,8 mg
Stéarate de magnésium : 1,2 mg
Eau purifiée q.s p. : 120 mg

Fomule d'enrobage :
Méthyl hydroxy propylcellulose 6 cps : 0,14 mg
Dioxyde de titane : 0,04 mg
Polyoxyéthylèneglycol 6000 : 0,02 mg
Eau purifiée : 1,8 mg
Talc pour un comprimé pelliculé q.s p. enrobé à : 122 mg

EXEMPLE 20

Forme injectable.
SR 44 709 : 1 mg
Polyoxyéthylène glycol 400 : 0,5 ml
Eau purifiée pour préparation injectable : qs pour 1 ml

EXEMPLE 21

Forme injectable.
SR 44 709 : 1 mg
Polysorbate 80[R] : 0,1 ml
Propylène glycol : 0,1 g
Eau purifiée pour préparation injectable : qs pour 1 ml.

Les produits selon l'invention ont été étudiés dans les tests de pharmacologie in vitro et in vivo A), B) et C) ci-dessous.

Comme composé de référence, on a utilisé le trans-cyano-6 (oxo-2 pyrrolidin-1-yl)-4 diméthyl 2,2 chromannol-3, décrit dans EP 76075:

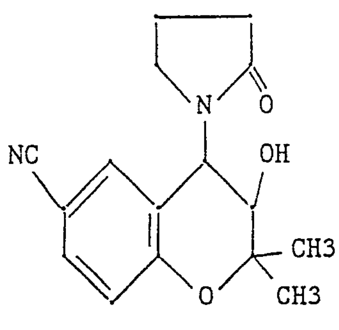

ci après désigné "Produit A", testé dans les mêmes conditions.

A) Veine porte isolée de rat.

Les rats mâles Sprague Dawley (250 - 300 g) sont assommés puis saignés après section de la gorge. La veine porte, ligaturée in situ en deux endroits distants de 15 mm, est isolée et coupée longitudinalement puis montée verticalement dans la cuve d'expérimentation contenant une solution physiologique à 37°C de composition suivante (mM) : NaCl : 137; KCl : 5,4; $MgCl_2$ : 1,05; $CaCl_2$ 1,8; $NaH_2PO_4$ : 1,2; $NaHCO_3$ : 15,5; Glucose 11,5 dans laquelle on fait barboter un mélange gazeux contenant 95 % d'oxygène et 5 % de gaz carbonique.

La veine est soumise à une tension de 500 mg. Après une période de stabilisation (environ 1 h 30) les activités contractiles spontanées sont enregistrées à l'aise d'un capteur isométrique. Chaque mesure est réalisée successivement sur 4 préparations.

Le produit est étudié à concentrations croissantes et successives (15 mm par concentration) jusqu'à

inhibition totale des contractions spontanées Les résultats sont exprimés sous forme de concentration molaire provoquant une inhibition de 50 pour cent (CI50) des activités contractiles spontanées.

Les résultats obtenus sont rassemblés dans le tableau 2 ci-dessous.

Dans la colonne des résultats, on a reporté la CI50 des activités contractiles spontanées de la veine porte isolée de rat.

TABLEAU 2

Inhibition des activités contractiles spontanées.

| Produit | Exemple | CI50 M |
|---|---|---|
| SR 44 709 | 1 | $9,0.\ 10^{-8}$ |
| SR 44 758 | 5 | $1,3.\ 10^{-7}$ |
| SR 44 793 | 4 | $3,4.\ 10^{-8}$ |
| SR 44 994 | 6 | $9,0.\ 10^{-9}$ |
| SR 45 012 | 11 | $2,6.\ 10^{-8}$ |
| SR 45 067 | 12 | $7,7.\ 10^{-8}$ |
| SR 45 135 | 13 | $8,0.\ 10^{-8}$ |
| SR 45 209 | 7 | $5,8.\ 10^{-8}$ |
| SR 45 218 | 8 | $2,0.\ 10^{-8}$ |
| SR 45 225 | 9 | $8,8.\ 10^{-8}$ |
| SR 45 311 | 10 | $5,0\ 10^{-8}$ |
| Produit A | - | $6,8.\ 10^{-8}$ |

Tous les composés étudiés présentent une importante activité inhibitrice des contractions spontanées de la veine, au moins du même ordre que le produit de référence. Quatre d'entre eux, SR 44 994, SR 44 793, SR 45 012 et SR 45 218, dont les CI50 sont égales ou inférieures à $3,4.\ 10^{-8}$, sont au moins 5 fois plus actifs que le produit A dans ce test.

B) Muscle papillaire de cobaye.

Les cobayes mâles Albinos Charles River (300 - 400 g) sont assommés puis saignés après section de la gorge Le coeur est isolé et ouvert, le muscle papillaire droit est excisé puis maintenu en survie dans la cuve d'expérimentation contenant une solution physiologique à 36°C (composition décrite ci-dessus).

La préparation est stimulée à l'aide d'une électrode bipolaire reliée à un stimulateur (fréquence = 60 battements par minute). Le potentiel d'action ventriculaire est recueilli selon la méthode classique de la microélectrode. Les paramètres caractéristiques ont été mesurés sur les potentiels d'action (PA) avant et après l'introduction du produit à tester à 3 concentrations successives et croissantes (30 minutes de perfusion par concentration). La concentration produisant une réduction de 50 pour cent de la durée du PA est indiquée (CI50).

Les résultats sont reportés dans le tableau 3 ci-dessous :

TABLEAU 3

Durée du potentiel d'action.

| Produit | Exemple | CI50 |
|---|---|---|
| SR 44 709 | 1 | $2,0.\ 10^{-5}$ |
| SR 44 793 | 4 | $2,7.\ 10^{-6}$ |
| SR 44 994 | 6 | $4,0.\ 10^{-6}$ |
| Produit A | - | $1,6.\ 10^{-5}$ |

Le tableau montre que la durée du potentiel d'action est nettement diminuée. Notamment, les concentrations de SR 44793 et SR 44994 produisant une réduction de 50 pour cent de ce paramètre sont 5 à 10 fois inférieures à celle du produit A, démontrant une activité électrophysiologique supérieure sur la perméabilité membranaire responsable de cette phase de repolarisation.

Le profil électrophysiologique des 3 composés étudiés est semblable: pas d'effet significatif sur le potentiel de repos et la vitesse maximale de dépolarisation, ceci signifie que les composés étudiés n'ont pas d'activité anesthésique locale.

C) Activité antihypertensive sur le rat spontanément hypertendu (SHR) vigile.

L'expérimentation est réalisée sur des rats SHR mâles (de souche Wistar) de 11 à 12 semaines; sous anesthésie au pentobarbital, un cathéter est implanté la veille de l'expérimentation dans une artère carotide. Lors de l'expérimentation les pressions artérielles diastolique (PAD) et systolique (PAS) des animaux vigiles sont enregistrées en continu 1 heure avant et jusqu'à 2 heures après l'administration du produit. La fréquence cardiaque (FC) est déterminée à partir de la pression artérielle pulsatile et enregistrée en continu pendant le même temps.

Les produits ont été administrés par voie orale sous un volume de 2 ml pour 100 g de poids d'animal après mise en suspension dans une solution aqueuse de gomme arabique à 5 %.

Les résultats sont reportés dans le tableau 4 ci-dessous.

TABLEAU 4

Diminution de la pression artérielle.

| Produit | Exemple | Dose mg/kg p.o. | Diminution maximale de la pression artérielle moyenne mm Mercure (± e.s.) |
|---|---|---|---|
| SR 44 709 | 1 | 0,06 | 21 ± 5 |
| | | 0,10 | 39 ± 12 |
| SR 44 758 | 5 | 0,03 | 12 ± 2 |
| | | 0,10 | 33 ± 5 |
| SR 44 793 | 4 | 0,03 | 22 ± 2 |
| | | 0,06 | 26 ± 3 |
| | | 0,10 | 50 ± 9 |
| SR 44 994 | 6 | 0,03 | 14 ± 0 |
| | | 0,10 | 37 ± 4 |
| Produit A | - | 0,03 | 16 ± 5 |
| | | 0,10 | 30 ± 10 |

e.s. = erreur standard

Les produits selon l'invention sont de puissants antihypertenseurs ayant une activité du même ordre que celle du Produit A.

Par contre, on a pu constater que deux des composés représentatifs de la présente invention, le SR 44 793 et le SR 44 994 possèdent une durée d'action plus longue que celle du Produit A.

Les produits de l'invention ont été également étudiés comme antiarythmiques dans le test D) ci-dessous.

D) Activite antiarythmique sur chien vigile.

La méthode utilisée est celle décrite par Dupuis et al., (Br. J. Pharmacol. 1976, 58, p. 409), dans laquelle un infarctus aigu est provoqué par l'insertion à thorax fermé d'une spire de cuivre dans la circulation coronaire.

L'électrocardiogramme est recueilli par télémétrie et les extrasystoles analysées et dénombrées automatiquement, tandis que l'animal fait l'objet d'une surveillance par circuit de télévision interne. Les produits ont été administrés par voie orale chez des animaux présentant au moins 50 pour cent d'extrasystoles.

Deux composés représentatifs de la présente invention, le SR 44 709 administré per os à 1 mg/kg et le SR 44 793 administré per os entre 0,1 et 0,5 mg/kg ont montré une importante activité antiarythmique en réduisant le nombre d'extrasystoles ou en restaurant un rythme sinusal pendant une durée variant de 2 heures à 10 heures selon les animaux.

Les données biologiques ci-dessus mettent en évidence que les composés selon l'invention sont des antihypertenseurs puissants et des antiarythmiques potentiels.

La toxicité aiguë d'un produit représentatif de l'invention, le SR 44 709, a été mesurée chez la souris per os aux doses de 10, 50, 500 et 1000 mg/kg pour un lot de 10 animaux, en comparaison avec le Produit A. Dans le cas du SR 44 709, tous les animaux sont restés en vie à la dose de 1000 mg/kg tandis que dans le cas du Produit A, les 10 animaux sont morts à la dose de 1000 mg/kg.

## Revendications

1. Dérivé du trans-diméthyl-2,2 chromannol-3 de formule :

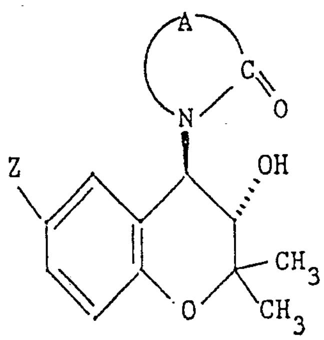

(I)

dans laquelle

- A, entre N et CO, représente le groupe - CH = CH - E = CH - ou le groupe

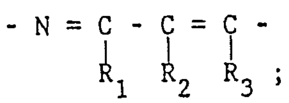

- Z représente un halogène ou un groupe cyano, acétyle, trifluoroacétyle, nitro, alkylthio, carboxy, phosphono, dialcoxyphosphoryle, alcoxycarbonyle, les groupes alkylthio et alcoxy contenant de 1 à 3 atomes de carbone ;
- E représente un atome d'azote ou un groupe C ($R_4$) ;
- $R_1$ représente l'hydrogène, un groupe méthyle ou un groupe hydroxyle, $R_2$ et $R_3$ représentent chacun indépendemment l'hydrogène ou un groupe méthyle, un seul des substituants $R_1$, $R_2$, $R_3$ pouvant être méthyle ;
- $R_4$ représente un atome d'hydrogène, d'halogène, un groupe méthyle ou un groupe hydroxyle ;

et les sels pharmaceutiquement acceptables du groupe phosphono ou carboxy.

2. Dérivé du trans-diméthyl-2,2 chromannol-3 selon la revendication 1 caractérisé en ce que Z représente le groupe cyano.

3. Le trans-cyano-6 (dihydro-1,2 oxo-2 pyridyl-1)-4 diméthyl -2,2 chromannol-3.

4. Le trans-cyano-6 (dihydro-1,2 hydroxy-4 oxo 2 pyridyl-1)-4 diméthyl-2,2 chromannol-3.

5. Le trans-cyano-6 (dihydro-1,6 hydroxy-3 oxo 6 pyridazinyl-1)-4 diméthyl-2,2 chromannol-3.

6. Procédé pour la préparation des composés selon l'une quelconque des revendications 1 à 5, caractérisé en ce que l'on traite un époxyde de chromanne de formule :

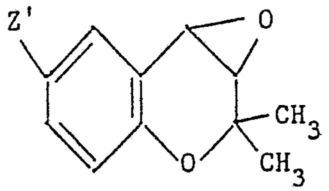

(II)

dans laquelle Z′ a la signification donnée dans la revendication 1 à Z, mais n'est pas le groupe carboxy ou le groupe phosphono, avec un hétérocycle de formule:

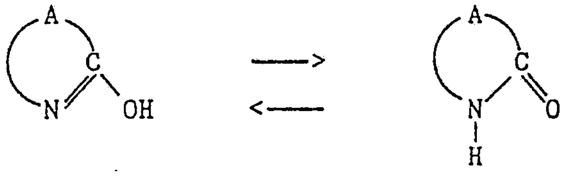

(III)

dans laquelle A a la signification indiquée dans la revendication 1;
on transforme éventuellement le groupe alcoxycarbonyle en groupe carboxy ou le groupe dialcoxyphosphoryle en groupe phosphono ; et on transforme éventuellement l'acide carboxylique ou l'acide phosphonique ainsi obtenu en l'un de leurs sels pharmaceutiquement acceptables.

7. Composition pharmaceutique caractérisée en ce qu'elle contient, comme principe actif, un dérivé de diméthyl-2,2 chromannol-3 selon l'une quelconque des revendications 1 à 5.

8. Composition pharmaceutique selon la revendication 7 contenant de 0,5 à 200 mg de principe actif par unité de dosage en mélange avec un excipient pharmaceutique.

9. Utilisation des dérivés du trans-diméthyl-2,2 chromannol-3 de formule :

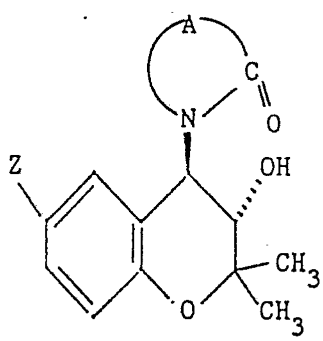

(I)

dans laquelle
- A, entre N et CO, représente le groupe - CH = CH - E = CH - ou le groupe

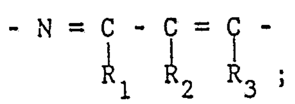

- Z représente un halogène ou un groupe cyano, acétyle, trifluoroacétyle, nitro, alkylthio, carboxy, phosphono, dialcoxyphosphoryle, alcoxycarbonyle, les groupes alkylthio et alcoxy contenant de I à 3 atomes de carbone ;
- E représente un atome d'azote ou un groupe C ($R_4$) ;
- $R_1$ représente l'hydrogène, un groupe méthyle ou un groupe hydroxyle; $R_2$ et $R_3$ représentent chacun indépendamment l'hydrogène ou un groupe méthyle, un seul des substituants $R_1$, $R_2$, $R_3$ pouvant être méthyle ;
- $R_4$ représente un atome d'hydrogène, d'halogène, un groupe méthyle ou un groupe hydroxyle ;
et les sels pharmaceutiquement acceptables du groupe phosphono ou carboxy, à la préparation de compositions pharmaceutiques à activités antiarythmique et antihypertensive.

**Revendications pour les Etats contractants suivants : ES et GR.**

1. Procédé pour l'obtention de dérivés du trans-diméthyl-2,2 chromannol-3 à activités antihypertensive et antiarythmique de formule :

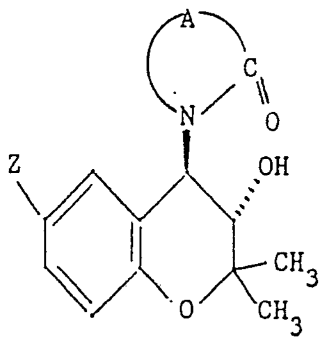

(I)

dans laquelle

- A, entre N et CO, représente le groupe - CH = CH - E = CH - ou le groupe

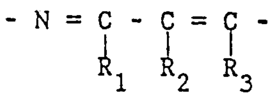

;

- Z représente un halogène ou un groupe cyano, acétyle, trifluoroacétyle, nitro, alkylthio, carboxy, phosphono, dialcoxyphosphoryle, alcoxycarbonyle, les groupes alkylthio et alcoxy contenant de 1 à 3 atomes de carbone ;
- E représente un atome d'azote ou un groupe C ($R_4$) ;
- $R_1$ représente l'hydrogène, un groupe méthyle ou un groupe hydroxyle; $R_2$ et $R_3$ représentent chacun indépendemment l'hydrogène ou un groupe méthyle, un seul des substituants $R_1$, $R_2$, $R_3$ pouvant être méthyle ;
- $R_4$ représente un atome d'hydrogène, d'halogène, un groupe méthyle ou un groupe hydroxyle ;

et les sels pharmaceutiquement acceptables du groupe phosphono ou carboxy, caractérisé en ce que l'on traite un époxyde de chromanne de formule :

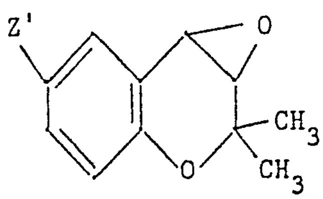

(II)

dans laquelle Z' a la même signification donnée ci-dessus à Z, mais n'est pas le groupe carboxy ou le groupe phosphono, avec un hétérocycle répondant à l'une des formes tautomères ci-après :

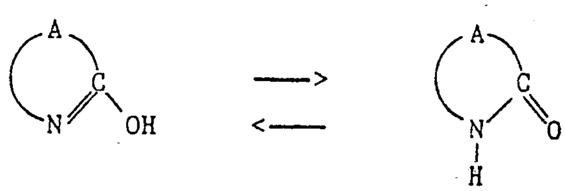

(III)

dans laquelle A a la signification indiquée ci-dessus ; on transforme éventuellement le groupe alcoxycarbonyle en groupe carboxy ou le groupe dialcoxyphosphoryle en groupe phosphono ; et on transforme éventuellement l'acide carboxylique ou l'acide phosphonique ainsi obtenu en l'un de leurs

sels pharmaceutiquement acceptables.

2. Procédé selon la revendication 1, caractérisé en ce que la première étape est réalisée à une température comprise entre 10° C et 100° C dans un solvant organique inerte en présence d'un agent de condensation basique.

3. Procédé selon l'une des revendications 1 ou 2, caractérisé en ce que l'on traite un époxyde de formule II, dans laquelle Z' est le groupe cyano.

4. Procédé selon l'une quelconque des revendications 1 à 3, caractérisé en ce que le composé de formule III est l'hydroxy-2 pyridine et en ce que le composé obtenu est le trans-cyano-6 (dihydro-1,2 oxo-2 pyridyl-1)-4 diméthyl-2,2 chromannol-3.

5. Procédé selon l'une quelconque des revendications 1 à 3, caractérisé en ce que le composé de formule III est le dihydroxy-2,4 pyridine et en ce que le composé obtenu est le trans-cyano-6 (dihydro-1,2 hydroxy-4 oxo-2 pyridyl-1)-4 diméthyl-2,2 chromannol-3.

6. Procédé selon l'une quelconque des revendications 1 à 3, caractérisé en ce que le composé de formule III est le dihydroxy-3,6 pyridazine et en ce que le composé obtenu est le trans-cyano-6 (dihydro-1,6 hydroxy-3 oxo-6 pyridazinyl-1)-4 diméthyl-2,2 chromannol-3.

7. Utilisation des dérivés du trans-diméthyl-2,2 chromannol-3 de formule :

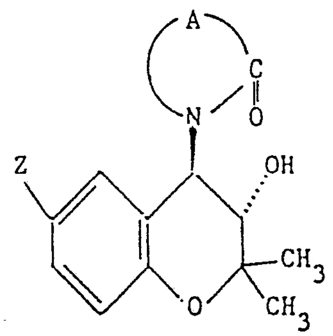

(I)

dans laquelle

- A, entre N et CO, représente le groupe - CH = CH - E = CH - ou le groupe

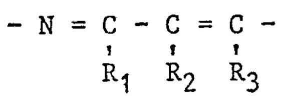

- Z représente un halogène ou un groupe cyano, acétyle, trifluoroacétyle, nitro, alkylthio, carboxy, phosphono, dialcoxyphosphoryle, alcoxycarbonyle, les groupes alkylthio et alcoxy contenant de 1 à 3 atomes de carbone :

- E représente un atome d'azote ou un groupe C ($R_4$) ;

- $R_1$ représente l'hydrogène, un groupe méthyle ou un groupe hydroxyle ; $R_2$ et $R_3$ représentent chacun indépendamment l'hydrogène ou un groupe méthyle, un seul des substituants $R_1$, $R_2$, $R_3$ pouvant être méthyle ;

- $R_4$ représente un atome d'hydrogène, d'halogène, un groupe méthyle ou un groupe hydroxyle ;

et les sels pharmaceutiquement acceptables du groupe phosphono ou carboxy, à la préparation de compositions pharmaceutiques à activités antihypertensive et antiarythmique.

Office européen des brevets

# RAPPORT DE RECHERCHE EUROPEENNE

Numero de la demande

EP 88 40 1580

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 076 075 (BEECHAM)<br>* Pages 5-10; page 19, ligne 20; revendications * | 1,2,6-9 | C 07 D 405/04<br>C 07 F 9/65<br>A 61 K 31/495<br>A 61 K 31/50 |
| A | EP-A-0 107 423 (BEECHAM)<br>* Pages 1-12; revendications * | 1,2,6-9 | |
| A | EP-A-0 172 352 (BEECHAM)<br>* Pages 2,12; page 21, exemple 3; revendications * | 1,2,6-9 | |

DOMAINES TECHNIQUES RECHERCHES (Int. Cl.4)

C 07 D 405/00
C 07 F 9/00
C 07 D 311/00

Le présent rapport a été établi pour toutes les revendications

| Lieu de la recherche | Date d'achèvement de la recherche | Examinateur |
|---|---|---|
| LA HAYE | 25-08-1988 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)